# EUROPEAN PATENT APPLICATION

(11) **EP 4 424 665 A1**
(43) Date of publication of application: **04.09.2024**
(21) Application number: 23159885.5
(22) Date of filing: 03.03.2023
(51) Int. Cl.: C07C 257/18, C07D 233/58, B01D 67/00, C08F 10/00

(54) **CATIONIC COMPOUNDS FOR ANION EXCHANGE MEMBRANES**

(71) Applicant: AGFA-GEVAERT NV, 2640 Mortsel (BE)
(72) Inventor: LOCCUFIER, Johan, 2640 Mortsel (BE); VAN DINGENEN, Wim, 2640 Mortsel (BE)
(74) Representative: Viaene, Kris

(57) **Abstract**

An amidinium-functionalized compound, characterized in that the compound has a structure according to General Formula I or General Formula II 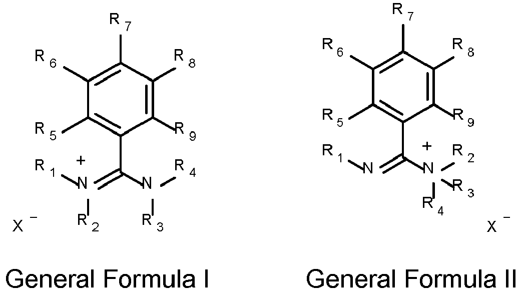 wherein
• R₅ and R₉ are any substituent different from hydrogen;
• R₁ to R₄ are independently selected from the group consisting of an alkyl group, an alkenyl group, an alkynyl group, an aralkyl group, an alkaryl group, an aryl group and a heteroaryl group, or any of R₁ and R₃, R₁ and R₄, R₁ and R₂, R₃ and R₄, R₂ and R₃, or R₂ and R₄ represent the necessary atoms to form a five- to eight- membered non-aromatic ring;
• R₆ to R₈ are independently selected from the group consisting of hydrogen, an alkyl group, an alkenyl group, an alkynyl group, an aralkyl group, an alkaryl group, an aryl or heteroaryl group, a halogen group, an ether group, a nitro group, an amine group, or any of R₅ and R₆, R₆ and R₇, R₇ and R₈, or R₈ and R₉ represent the necessary atoms to form a five-to eight-membered ring;
• X⁻ is an anion;
and wherein
• at least one of R₁ to R₉ comprises a polymerizable group or comprises the necessary atoms to link the amidinium group to a polymer.

## Description

### Technical Field

The present invention relates to the field of anion exchange membranes, more specifically to cationic monomers and polymers for the production thereof. The invention has potential applications in water electrolysis.

### Background Art

To lead future generations towards a climate-neutral world, alternatives to fossil fuels are becoming more and more important. Renewable energy is generated from natural sources that can be replenished over time, such as solar, wind, hydropower, geothermal, bioenergy, and tidal/wave energy. Water electrolysis is one of the technologies that can be used to exploit these renewable energies, thereby producing green hydrogen for a sustainable future. Hydrogen gas is an efficient energy carrier and storage medium and can be an alternative to fossil fuels to generate power.

Although alkaline water electrolysis (AWE) is the established technology for hydrogen production, the combination with renewable energy poses a challenge because of the intermittent availability inevitably linked to this kind of energy. Intermittent power supplies result in fluctuations in voltage and current, leading to increased gas cross-over during the electrolysis process: problems which are associated with the porous structure of the AWE separator.

Proton Exchange Membrane (PEM) water electrolysis is a promising alternative to AWE, but its commercialization is limited by the necessity to use expensive platinum and iridium catalysts. Anion Exchange Membrane (AEM) water electrolysis on the other hand combines the use of cheap and abundant catalyst materials with the advantages of PEM water electrolysis.

Specifically for water electrolysis, the use of AEMs has a range of benefits compared to PEM and AWE, such as the relatively low cost, resistance to pressure, a large operational capacity, and a fast response to changing operating conditions. An AEM should be a thin, stable, gas tight and highly conductive polymeric membrane. Typically, an AEM is a cationic polyelectrolyte which transfers charges over the membrane by hydroxide ions. The cationic groups can be present as pendent functionality or as a part of the polymeric main chain. Examples of cationic groups are quaternary ammonium, guanidinium, imidazolium, benzimidazolium, phosphonium, and cobaltocenium groups. Due to their ion exchange capacity, AEMs are not only applied in water electrolysis, but also in (waste) water treatment, electro-dialysis, chlor-alkali production, desalination processes, batteries, and fuel cells.

WO2019076860 (EVONIK) discloses polymeric compounds containing benzimidazolium units for anion exchange membranes and WO2021013694 (EVONIK) describes polymeric compounds containing a spiro or piperidine structural unit as anion conducting membranes. The membranes have a high mechanical stability and low swelling characteristics, as well as a quite high anion conductivity.

One specific problem in AEMs is their low alkaline stability, resulting from a degradation reaction occurring at the polymer backbone or at the functional group. While degradation at the backbone results in mechanical defects of the membrane, degradation at the functional group reduces the ion conductivity. Degradation mostly occurs by a nucleophilic attack by hydroxide ions, which can destroy the anion-exchange capacity and hydroxide ion conductivity.

WO2022207470 (FUJIFILM) discloses a cationically charged polymeric membrane comprising a cationically charged nitrogen atom in an aromatic heterocyclic ring, resulting in a high ion-exchange capacity, combined with a low electrical resistance, even after being exposed to harsh conditions.

WO2017117678 (IONOMR) and WO20188023097 (IONOMR) describe a cationic membrane comprising a more alkaline-stable cationic group that can be used in anion exchange resins, based on methylated polybenzimidazoles, sterically hindered by the introduction of methyl groups in both ortho-positions of the linking 2-phenyl group.

However, there is still a need to further improve the alkaline stability of AEMs, to prevent chemical and mechanical degradation, thereby increasing their durability.

### Summary of invention

It is an object of the invention to provide a cationic monomer or polymer that has an improved stability under alkaline conditions.

This object is realised by the amidinium-functionalized compound as defined in claim 1.

It is a further object of the invention to provide a method for the preparation of such cationic polymer.

Further objects of the invention will become apparent from the description hereinafter.

### Description of embodiments

### Definitions

The term "monofunctional" in e.g. monofunctional polymerizable compound means that the polymerizable compound includes one polymerizable group.

The term "difunctional" in e.g. difunctional polymerizable compound means that the polymerizable compound includes two polymerizable groups.

The term "polyfunctional" or "multifunctional" in e.g. polyfunctional polymerizable compound means that the polymerizable compound includes more than two polymerizable groups.

The term "alkyl" means all variants possible for each number of carbon atoms in the alkyl group i.e. methyl, ethyl, for three carbon atoms: n-propyl and isopropyl; for four carbon atoms: n-butyl, isobutyl and tertiary-butyl; for five carbon atoms: n-pentyl, 1,1-dimethyl-propyl, 2,2-dimethylpropyl and 2-methyl-butyl, etc.

Unless otherwise specified a substituted or unsubstituted alkyl group is preferably a C₁ to C₆-alkyl group.

Unless otherwise specified a substituted or unsubstituted alkenyl group is preferably a C₂ to C₆-alkenyl group.

Unless otherwise specified a substituted or unsubstituted alkynyl group is preferably a C₂ to C₆-alkynyl group.

Unless otherwise specified a substituted or unsubstituted alkaryl group is preferably a phenyl or naphthyl group including one, two, three or more C₁ to C₆-alkyl groups.

Unless otherwise specified a substituted or unsubstituted aralkyl group is preferably a C₇ to C₂₀-alkyl group including a phenyl group or naphthyl group.

Unless otherwise specified a substituted or unsubstituted aryl group is preferably a phenyl group or naphthyl group

Unless otherwise specified a substituted or unsubstituted heteroaryl group is preferably a five- or six-membered ring substituted by one, two or three oxygen atoms, nitrogen atoms, sulphur atoms, selenium atoms or combinations thereof.

The term "substituted", in e.g. substituted alkyl group means that the alkyl group may be substituted by other atoms than the atoms normally present in such a group, i.e. carbon and hydrogen. For example, a substituted alkyl group may include a halogen atom or a thiol group. An unsubstituted alkyl group contains only carbon and hydrogen atoms.

Unless otherwise specified a substituted alkyl group, a substituted alkenyl group, a substituted alkynyl group, a substituted aralkyl group, a substituted alkaryl group, a substituted aryl and a substituted heteroaryl group are preferably substituted by one or more constituents selected from the group consisting of methyl, ethyl, n-propyl, isopropyl, n-butyl, isobutyl and tertiary-butyl, ester, amide, amine, ether, thioether, ketone, aldehyde, sulfoxide, sulfone, sulfonate ester, sulphonamide, -Cl, -Br, -I, -OH, -SH, - CN and -NO₂.

### Amidinium-functionalized compound

The amidinium-functionalized compound according to the present invention has a structure according to General Formula I or General Formula II 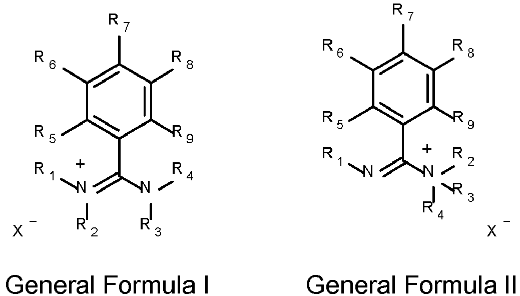 wherein
- R₅ and R₉ are any substituent different from hydrogen;
- R₁ to R₄ are independently selected from the group consisting of a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group, or any of R₁ and R₃, R₁ and R₄, R₁ and R₂, R₃ and R₄, R₂ and R₃, or R₂ and R₄ represent the necessary atoms to form a five- to eight- membered non-aromatic ring;
- R₆ to R₈ are independently selected from the group consisting of hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group, a substituted or unsubstituted aryl or heteroaryl group, a halogen, an ether, a nitro, and an amine, or any of the substituents R₅ and R₆, R₆ and R₇, R₇ and R₈, or R₈ and R₉ represent the necessary atoms to form a fiveto eight-membered ring;
- X- is an anion to counterbalance the cationic charge;
and wherein
- at least one of R₁ to R₉ comprises a polymerizable group or comprises the necessary atoms to link the amidinium group to a polymer.

An AEM typically comprises a positively charged polymer containing cationic groups such as ammonium groups, imidazolium groups, guanidinium groups and phosphonium groups, allowing transportation of anions, for example hydroxide ions, through the membrane. One of the major challenges in the design of AEMs is to overcome the low alkaline stability, in part resulting from dealkylation reactions occurring at these cationic groups.

The compound according to the present invention comprises an amidinium group as cationic group. An amidinium group is a cationic group obtained by quaternizing an amidine group RC(NR′)NRʺR‴, where R, R', R", and R‴ can be the same or different. Amidines are the imine derivatives of amides. The amidinium group has a higher pKa of the corresponding base compared to the corresponding bases of the above-listed typically used cationic groups. It is believed that this could reduce dealkylation reactions and therefore result in a higher alkaline stability.

To increase the alkaline stability of the compound even further, the amidinium-functionalized compound according to the present invention comprises groups to sterically protect the amidinium group from nucleophilic attack at the cation. To optimally protect the amidinium group, the protective groups are introduced at both ortho-positions of the 2-phenyl group: R₅ and R₉. Therefore, R₅ and R₉ are any substituent different from hydrogen. R₅ and R₉ are preferably an alkaline-stable substituent, which is defined as a functional group showing less than 5% degradation when treated in 2M KOH for 7 days at 80 °C. More preferably, the degradation is less than 2% and most preferably, there is no degradation detectable.

Without being limited thereto, R₅ and R₉ may be independently selected from the group consisting of a substituted or unsubstituted alkyl group, a substituted or unsubstituted alkenyl group, a substituted or unsubstituted alkynyl group, a substituted or unsubstituted aralkyl group, a substituted or unsubstituted alkaryl group, a substituted or unsubstituted aryl or heteroaryl group, a halogen, an ether group, a nitro group, and an amine group.

Preferably, R₅ and R₉ are independently selected from the group consisting of a substituted or unsubstituted alkyl group, a substituted or unsubstituted aryl group, a halogen, and an ether group. More preferably, R₅ and R₉ are independently selected from a substituted or unsubstituted alkyl group and a substituted or unsubstituted aryl group, most preferably R₅ and R₉ are independently selected from an unsubstituted C1 to C4 alkyl group and a substituted or unsubstituted phenyl group, a phenyl group and methyl group being particularly preferred. When R₅ and R₉ are independently from each other a methyl or a phenyl group, the amidinium group is sufficiently sterically protected against nucleophilic attack, while not substantially sterically hindering the synthesis of the desired amidinium-functionalized compound, described below.

The amidinium-functionalized compound according to the invention may be a monomer or a polymer. When at least one of R₁ to R₉ comprises a polymerizable group, the amidinium-functionalized compound is referred to as an amidinium-functionalized monomer, which is described in more detail below. When at least one of R₁ to R₉ comprises the necessary atoms to link the amidinium group to a polymer, the amidinium-functionalized compound is referred to as an amidinium-functionalized polymer, which is described in more detail below.
In a preferred embodiment, at least one of R₁, R₂, R₃, R₄ and R₇ comprises a polymerizable group, as disclosed further. In another preferred embodiment, at least one of R₁, R₂, R₃, R₄ and R₇ comprises the necessary atoms to link the amidinium group to a polymer backbone. In a further preferred embodiment, at least two of R₁, R₂, R₃, R₄ and R₇ are integrated into a polymer backbone. When at least two of R₁, R₂, R₃, R₄ and R₇ are integrated into the polymer backbone, this results in the amidinium group also being part of the polymer backbone. The structure of the amidinium-functionalized polymer according to the present invention is discussed further in more detail.

Preferably, R₆ to R₈ are independently selected from the group consisting of a hydrogen, a substituted or unsubstituted alkyl group, and a substituted or unsubstituted aryl group. More preferably, R₆ to R₈ are selected from a hydrogen and a C1 to C6 alkyl group, most preferably from a hydrogen and a methyl group.

The amidinium-functionalized compounds of the present invention contain an anion X- to counterbalance the cationic charge of the compound. Without being limited thereto, X- is selected from the group consisting of iodide, bromide, chloride, fluoride, triiodide, hydroxide, carbonate, bicarbonate, cyanide, acetate, nitrate, sulfate, alkyl sulfate, perfluorinated alkylsulfonate, arylsulfonate, perchlorate, tetrachloroaluminate, tetrafluoroborate, alkyl borate, phosphate, halophosphate, alkyl phosphate, triflate, tosylate, mesylate, alkyl carboxylate, tetrakis(3,5-bis(trifluoromethyl)phenyl)borate, bis(trifluoromethane)sulfonamide, and any combination thereof.

A preferred anion is selected from the group of consisting of bromide, chloride, hydroxide, benzene sulfonate, tosylate, and mesylate.

The amidinium-functionalized compounds according to the present invention are preferably prepared according to the synthetic strategy described below: 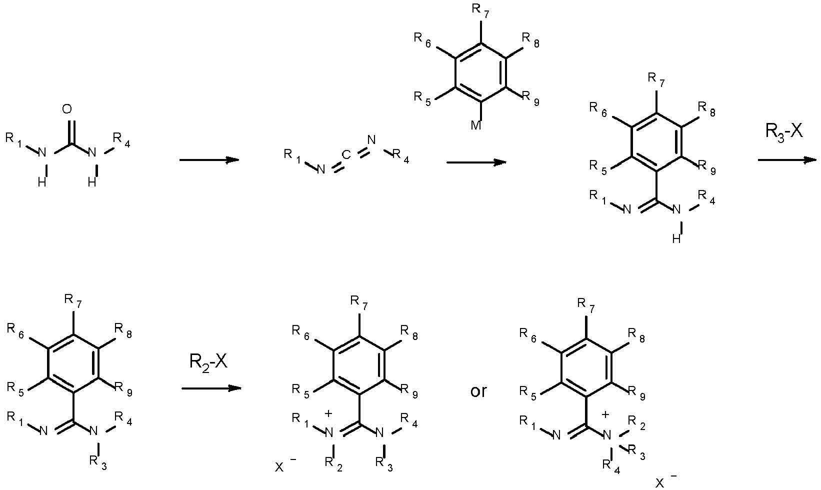 wherein R₁ to R₉ are as defined above and M represents a metal atom, optionally linked to a halide atom.

In a first step, a substituted urea R₁HNCONHR₄ or thiourea R₁HNCSNHR₄ is converted into its corresponding carbodiimide using any method known in literature, followed by addition of a sterically hindered aromatic organometallic reagent, having R₅ and R₉ as hindering groups. Preferably, aryl lithium and Grignard reagents are used as organometallic reagents. Preferred sterically hindered aromatic organometallic reagents are: (2,4,6-trimethylphenyl)magnesium bromide, (2,4,6-trimethylphenyl)magnesium chloride, (2,4,6-trimethylphenyl) lithium, (2,6-dimethylphenyl) magnesium bromide, (2,6-dimethylphenyl)lithium, (2,3,4,5,6-pentamethylphenyl)magnesium bromide, (2,3,4,5,6-pentamethylphenyl)lithium, (4-methoxy-2,6-dimethylphenyl)magnesium bromide, (4-methoxy-2,6-dimethylphenyl)lithium, (2,6-dimethoxyphenyl)magnesium bromide, (2,6-dimethoxyphenyl)lithium, (2,4,6-trimethoxyphenyl)magnesium bromide, (2,4,6-trimethoxyphenyl)lithium, (2,6-diphenylphenyl)magnesium bromide, (2,6-diphenlyphenyl)lithium, (2,4,6-triphenylphenyl)magnesium bromide and (2,4,6-triphenylphenyl)lithium.

The obtained amidine is then alkylated with a first alkylation reagent R₃-X, followed by quaternization with a second alkylation agent R₂-X. R₂ and R₃ are as defined above and can be the same or different. An alkylation reagent is also referred to as alkylating agent. Suitable alkylating agents are alkyl halides, such as bromides, iodides, chlorides, and fluorides. Other typical alkylating agents are tosylates, mesylates, and benzenesulfonates.

### Amidinium- functionalized compound comprising a polymerizable group

According to one embodiment of the invention, the amidinium-functionalized compound is an amidinium-functionalized monomer. In this case, at least one of R₁ to R₉ comprises a polymerizable group. Preferably, at least one of R₁, R₂, R₃, R₄ or R₇ comprises a polymerizable group, selected from the group consisting of styrene, epoxy, oxetane, vinyl ether and alkene. Preferred polymerizable groups are styrene groups, vinyl ether groups, and alkene groups. Most preferably, the polymerizable group is a styrene group.

The amidinium-functionalized monomers may comprise one or more polymerizable groups. Preferably, the amidinium-functionalized monomers are monofunctional, i.e. they comprise a polymerizable group.

The amidinium-functionalized monomers are preferably prepared starting from an amidine-functionalized monomer as a precursor compound. This amidine-functionalized monomer can be converted in an amidinium-functionalized monomer by quaternization with a suitable alkylation agent. A particularly preferred strategic route to obtain an amidinium-functionalized monomer is as described above.

Without being limited thereto, examples of monomers according to the present invention are listed in Table 1.

**Table 1**

| | |
|---|---|
| 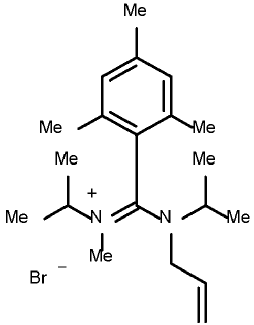 | AM-1 |
| 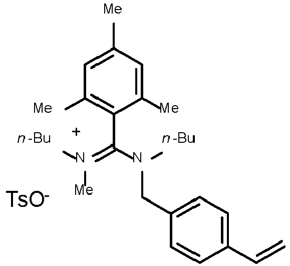 | AM-2 |
| 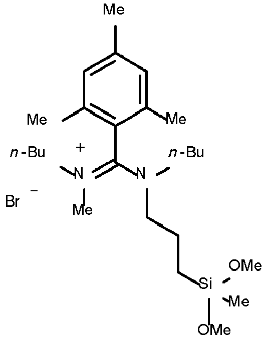 | AM-3 |
| 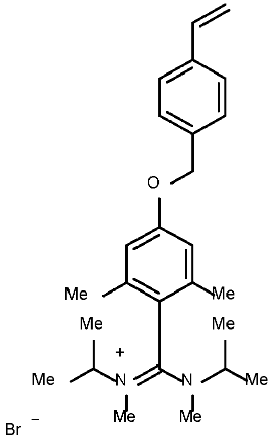 | AM-4 |
| 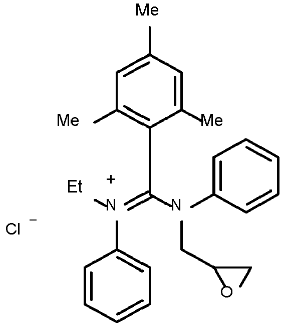 | AM-5 |
| 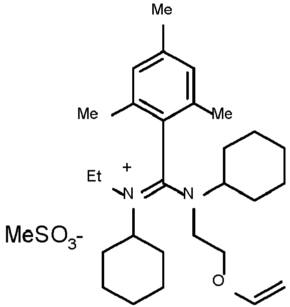 | AM-6 |
| 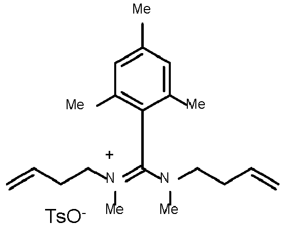 | AM-7 |
| 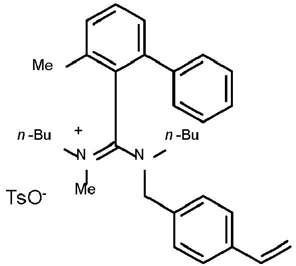 | AM-8 |
| 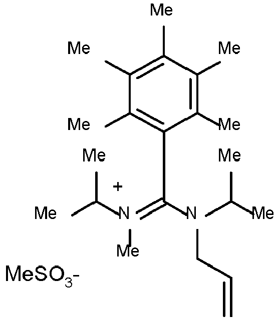 | AM-9 |
| 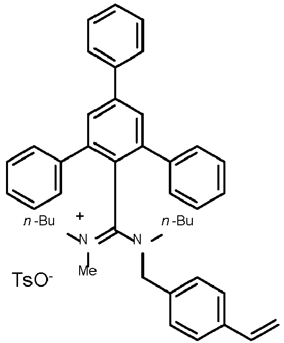 | AM-10 |
| 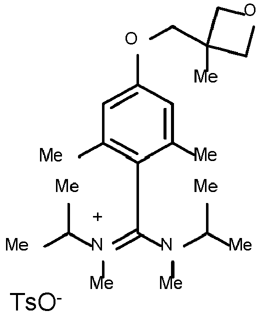 | AM-11 |
| 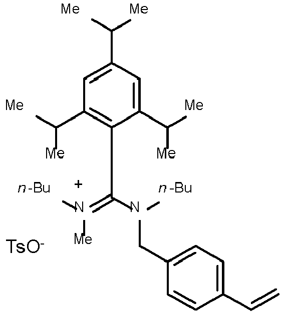 | AM-12 |
| 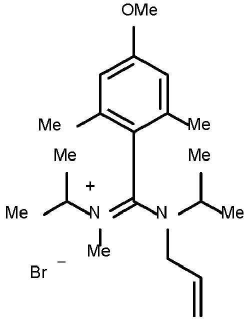 | AM-13 |
| 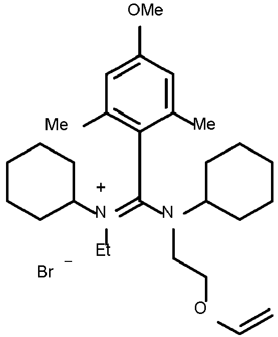 | AM-14 |
| 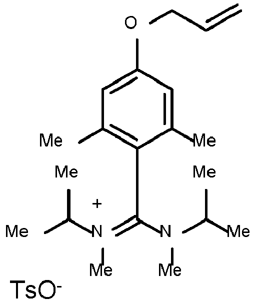 | AM-15 |

### Amidinium-functionalized polymer

According to another embodiment of the invention the amidinium-functionalized compound is an amidinium-functionalized polymer. In this case, at least one of R₁ to R₉ comprises the necessary atoms to link the amidinium group to a polymer. Preferably, at least one of R₁, R₂, R₃ R₄, and R₇ comprises the necessary atoms to link the amidinium group to a polymer backbone

In another preferred embodiment, at least two of R₁, R₂, R₃ R₄, and R₇ are integrated into a polymer backbone. When at least two of R₁, R₂, R₃, R₄ and R₇ are integrated into a polymer backbone, this results in the amidinium group being part of the polymer backbone.

More preferably, the polymer according to the present invention comprises a monomeric unit of the amidinium-functionalized monomer described above. This means that the polymer according to the present invention comprises a monomeric unit that is derived from the amidinium-functionalized monomer described above. A monomeric unit is defined as the largest constitutional unit contributed by a single monomer molecule to the structure of a macromolecule (IUPAC). If the polymer is a homopolymer, the monomeric unit may also be referred to as a repeating unit.

The amidinium-functionalized polymer according to the present invention can be any type of polymer. The polymer can be a linear polymer, a star shaped polymer, a crosslinked polymer, or a (hyper)branched polymer.

The amidinium group can be an integrated part of the polymer backbone, also referred to as polymer main chain, or can be part of a side chain covalently linked to the polymer backbone. The definition of the polymer main chain or backbone is known to the person skilled in the art and can be defined as: "That linear chain to which all other chains, long or short or both, may be regarded as being pendant." (IUPAC definition). For example, polyolefins such as polyethylene, polypropylene, polystyrene, and acrylates have main chains composed of carbon: ...C-C-C-C... The polymer side chain is the chemical group that is attached to the main chain. The side chain may be short, oligomeric, or polymeric.

The amidinium group can be an integrated part of the polymer backbone, which means that at least one nitrogen atom of the amidinium group is integrated in the polymer main chain. The integration of the amidinium group in the polymer main chain could have the advantage that the mechanical properties of the AEM are further improved, by further reducing degradation reactions. Another benefit could be a better ion conductivity. Preferably, both nitrogen atoms of the amidinium group are incorporated in the polymer main chain.

The amidinium group can also be a part of a side chain of the polymer backbone. This has the advantage that the polymer backbone can be designed in such a way as to further increase the alkali stability or to tune the mechanical properties of the AEM.

The amidinium-functionalized polymer can be selected from an amidinium-functionalized polyolefin, polyethylene, polyacrylate, polymethacrylate, polystyrene, polysulfone, poly(phenylene oxide), poly(phenylene), poly(benzimidazolium), poly(arylene ether ketone), polyarylene or poly(arylene ether sulfone), polyetheretherketone, polyimide or a polyamide. Preferably, the amidinium-functionalized polymer is an amidinium-functionalized polystyrene.

Any polymerization method can be used to prepare the polymers according to the present invention, including free radical polymerization, living free radical polymerization, ring opening polymerization, cationic polymerization, anionic polymerization, metathesis polymerization, Ziegler-Natta polymerization, polyaddition and polycondensation. Preferred polymerization techniques are free radical polymerization and living free radical polymerization.

Any type of suitable initiator may be used to initiate the polymerization reaction. For radical polymerization processes, any radical polymerization initiator may be used, such as redox initiators, photoinitiators or thermal initiators. Preferred initiators are thermal polymerization initiators, which generate radicals upon exposure to heat. Examples of thermal polymerization initiators are organic peroxides, inorganic peroxides, and azo initiators, of which azobisisobutyronitrile (AIBN) is the most common example. Other azo initiators, such as for example 2,2'-azobis(2-(2-imidazolin-2-yl) propane) dihydrochloride; 2,2'-azobis(N,N'-dimethylene isobutyramidine) dihydrochloride; 2,2'-azobis-(amidinopropane) dihydrochloride; 2,2'-azobis(2-methylpropionamidine)dihydrochloride); 2,2'-azobis (2,4 dimethylvaleronitrile); 2,2'-azobis(2-methylbutyronitrile); or dimethyl 2,2'-azobis(2-methylpropionate) are commercially available from WAKO Chemicals or Vesta Chemicals.

A preferred amount of radical initiator is from 0.02 to 20 mol%, more preferably from 0.1 to 10 mol%, most preferably from 0.5 to 5 mol%, relative to the total amount of the monomer.

The polymers according to the present invention can also be obtained by post-derivatization methods on an existing polymer backbone. A preferred post-derivatization method comprises the quaternization of an amidine-functionalized precursor polymer, using an alkylating agent such as for example an alkyl tosylate. The amidine-functionalized polymer can be prepared by the polymerization of an amidine-functionalized monomer, preferably using free radical polymerization, or living free radical polymerization techniques such as described above. The amidine-functionalized precursor monomer can be prepared as described above.

The polymers according to the present invention can be homopolymers or co-polymers comprising two or more monomers. Without being limited thereto, the co-polymers can be random co-polymers, block co-polymers, alternating co-polymers, graft co-polymers, gradient co-polymers, and comb or comb-like co-polymers.

Co-polymers are typically prepared from a mixture of monomers, in a predetermined ratio. Depending on the desired properties, different ratios of monomers can be used. It is also possible to add the monomers at different times of the polymerization process to obtain a block co-polymer or a gradient co-polymer. Block or block-like co-polymers exhibit different characteristics compared to random co-polymers. The amidinium-functionalized monomer can be combined with any other type of monomer. A mixture of different amidinium-functionalized monomers can be used. The amidinium-functionalized monomer can be co-polymerized with a second type of amidinium-functionalized monomer or with another cationically functionalized monomer, such as a guanidinium-functionalized monomer, a benzimidazolium-functionalized monomer, or an ammoniumfunctionalized monomer. Co-polymers can also be prepared by copolymerizing the amidinium-functionalized monomer with a nonionic monomer, such as for example styrene. A preferred amount of cationically functionalized monomer in a co-polymer is from 10 to 90 mol%, more preferably from 20 to 80 mol%, most preferably from 30 to 70 mol%, compared to the total mols of monomer.

In another aspect of the invention, the amidinium-functionalized polymer according to the present invention is provided in a dispersed form as a polymer dispersion. This polymer dispersion can be mixed with catalyst which, in turn, can be deposited on substrates, such as metallic porous structures and membranes. Following removal of the liquid medium, the polymer will act as binder of the catalyst onto the substrate (see further).

A polymeric dispersion can be provided both in aqueous and in solvent medium. Preferably, the polymer dispersion is an aqueous dispersion or is provided in an alcohol, such as ethanol. Preferably, the dispersion contains from 2.5 to 70 wt%, more preferably from 10 to 50 wt%, most preferably from 5 to 25 wt% of amidinium-functionalized polymer.

The amidinium-functionalized polymers according to the present invention can be self-dispersing by electrostatic forces or can be dispersed by means of a dispersing agent, which can be a polymeric dispersant or a non-polymeric surfactant. Dispersing agents can be non-ionic, cationic, or anionic. Since the amidinium-functionalized polymer is cationic in nature, a cationic or non-ionic dispersing agent is preferred. Suitable dispersants are DISPERBYK^{™} dispersants available from BYK CHEMIE, JONCRYL^{™} dispersants available from JOHNSON POLYMERS and SOLSPERSE^{™} dispersants available from Lubrisol. Another example is poly(diallyldimethylammonium chloride), also known as polyDADMAC. A detailed list of non-polymeric as well as some polymeric dispersants is disclosed by MCCUTCHEON. Functional Materials, North American Edition. Glen Rock,N.J.: Manufacturing Confectioner Publishing Co., 1990. p.110-129.

The polymeric dispersion can be obtained using any type of technique, such as solvent evaporation, milling, emulsion polymerization, mini-emulsion polymerization, or dispersion polymerization. Preferred dispersing techniques are solvent evaporation and mini-emulsion polymerization.

Typical polymers and co-polymers according to the present invention are given in Table 2 below, without being limited thereto. The structures are general structures, and the ratio of co-monomers can vary according to the desired properties. The co-polymers can be random, block or gradient co-polymers.

| | |
|---|---|
| 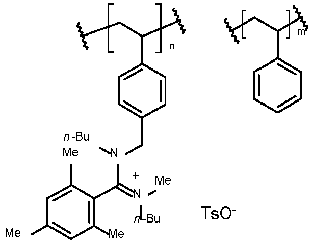 | AP-1 |
| 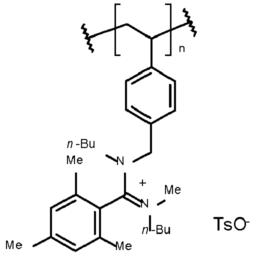 | AP-2 |
| 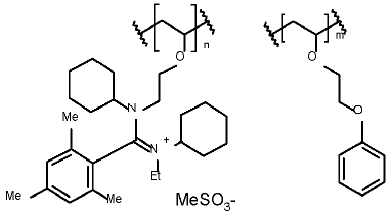 | AP-3 |
| 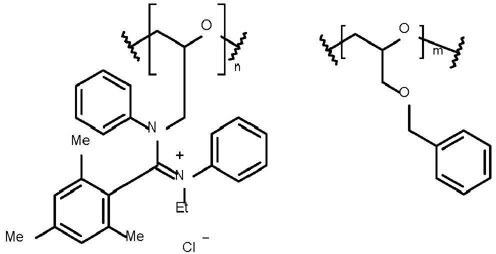 | AP-4 |
| 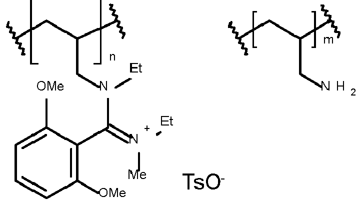 | AP-5 |
| 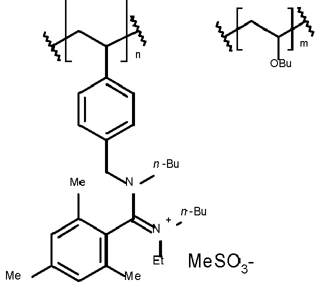 | AP-6 |
| 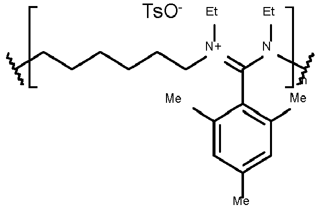 | AP-7 |

### Anion-exchange membrane

The amidinium-functionalized polymers according to the present invention can be used for any application for which a cationic polymer is necessary, e.g., as a flocculant in (waste) water treatment, or as an additive in textile finishing. Preferably, the amidinium-functionalized polymers are used to produce anion-exchange membranes (AEMs).

AEMs allow for the transportation of anions (e.g. OH-, Cl-, Br) from the cathode to the anode in an electrochemical reaction. They can be used in any electrochemical process, selected from electrolysis, electrodialysis and fuel cell technology. Preferably, the anion-exchange membranes in the present invention are used for electrolysis, more preferably for water electrolysis. In water electrolysis, hydroxide ions (OH-) are transported through the AEM, along with water molecules. The AEMs according to the present invention can also be used in batteries, sensors, and actuators.

There is an important balance to be found between mechanical properties and conductivity of the AEM. Preferably, the AEM has a long lifespan, which can be achieved by membranes having good mechanical properties. A thicker membrane will typically improve the mechanical properties of the AEM and will reduce hydrogen and oxygen cross-over. The AEM membrane according to the present invention preferably has a dry thickness of 10 to 100 µm, more preferably from 20 to 80 µm, most preferably from 30 to 60 µm. When the AEM is wet, the membrane may swell and the thickness can increase.

However, a too high thickness will result in a higher ionic resistance and thereby reduce the ion conductivity of the AEM. A high anion conductivity is required to support large currents with minimal resistive losses. The anion conductivity of the AEM according to the present invention is preferably more than 40 mS/cm, more preferably more than 70 mS/cm, most preferably more than 100 mS/cm.

Not only the thickness of the AEM determines the mechanical properties, also the chemical structure of the used polymer, the crosslinking degree, and optional additives have an impact.

When the AEM comprises a chemically or physically crosslinked polymer, it preferably has a swelling ratio of 5 to 60%, more preferably, it has a swelling ratio of 10 to 50%, most preferably it has a swelling ratio of 20 to 40%. When the swelling ratio is lower, often the mechanical properties of the AEM are better.

The strength of the AEM is represented by its tensile strength. The tensile strength of the AEM according to the present invention is preferably from 10 to 50 MPa. More preferably, the tensile strength is from 12 to 40 MPa, most preferably from 15 to 34 MPa.

The anion-exchange membranes can be produced by any method known in the art, depending on the desired size and scale. Generally, any coating or casting technique can be used. Preferred techniques are slot dye coating, extrusion coating, and dip coating. A preferred method for making an AEM on a small scale is solution casting of a polymer solution and evaporating the solvent. Evaporation of the solvent can be facilitated by heating or by applying a vacuum, or a combination thereof. Another preferred method for making an AEM consists of coating of the polymer solution using a bar coater and coating knife. The processes can be performed under air or under nitrogen atmosphere.
A preferred large-scale production method for AEMs is slot dye coating.

The polymer solution used to prepare the AEM can be a solution of amidinium-functionalized polymer, obtained directly after the polymer synthesis. But when a higher purity is desired, it is also possible to isolate and purify the amidinium-functionalized polymer after its synthesis, and to re-dissolve it in a solvent of choice, such as for example propylene glycol, NMP, NBP, DMSO, DMF, THF, MEK, and dioxolane. In principle, any solvent can be used in which the polymer is soluble. It is also possible to dissolve the polymers in a mixture of solvents or in water-solvent mixtures. Preferred mixtures are water-isopropanol and water-ethanol. Preferably, the solvent or mixture of solvents has a boiling point below 175 °C.

It is also possible to manufacture the AEM using a solution of an amidine-functionalized precursor polymer, which is afterwards quaternized. In a first step, a non-ionic membrane is thus formed, which is then reacted with a suitable alkylating agent, to obtain a cationic polymer membrane.

It is further possible to crosslink the polymer to increase the mechanical strength of the membrane. Any type of crosslinking reaction can be used, but preferred methods include thermal curing, photocuring, electron beam irradiation, gamma irradiation, and combinations thereof. It is further possible to cure the polymer in two separate steps, which means that both a UV-crosslinking step and a thermal curing step are carried out consecutively, without being limited to a certain order of steps.

The anion exchange membrane according to the present invention can comprise a porous or a non-porous support, also referred to as a reinforcing support. Without being limited thereto, the support can be selected from expanded polymer films such as expanded PTFE, and polymer wire meshes, which can be woven or non-woven. An AEM comprising a support has the advantage that the mechanical properties are further improved.

The process for preparing the anion-exchange membrane having a support preferably comprises impregnating or coating the support with a solution containing the polymer. Optionally, a curing reaction may be performed to cross-link the polymer and thus create an even more durable AEM.

### Electrochemical device comprising anion-exchange membrane

The AEM according to the present invention may be used in any electrochemical device, such as an electrolyser, a redox-flow battery, a fuel cell, or an electrodialysis system.

The AEM according to the present invention is preferably used in a water electrolyser.

An electrolysis cell typically comprises two electrodes, an anode, and a cathode, separated by a membrane. An electrolyte is present between both electrodes.

When electrical current is supplied to the electrolysis cell, hydroxyl ions of the electrolyte are oxidized into oxygen at the anode and water is reduced to hydrogen at the cathode. The hydroxyl ions formed at the cathode migrate through the membrane to the anode. The membrane limits transfer of hydrogen and oxygen formed during water electrolysis, from cathode to anode and from anode to cathode respectively.

The AEM water electrolyser can be operated in pure water. The AEM water electrolyser can also be operated in an electrolyte solution, which is typically an alkaline solution. Preferred electrolyte solutions are aqueous solutions of electrolytes selected from sodium hydroxide or potassium hydroxide. Potassium hydroxide electrolytes are often preferred due to their higher specific conductivity. The concentration of the electrolyte in the electrolyte solution is preferably between 0.01 mol/L and 2 mol/L. Consequently, the pH of the electrolyte solution is between 7 and 14, more preferably between 7 and 12.

The temperature of the electrolyte solution is preferably from 35 °C to 100 °C, more preferably from 40 °C to 80 °C, most preferably from 45 to 60 °C.

An electrode typically includes a substrate provided with a so-called catalyst layer. The catalyst layer can be the same or different for the anode, where oxygen is formed, and the cathode, where hydrogen is formed.

Typical electrode substrates are made from electrically conductive materials selected from the group consisting of nickel, iron, soft steels, stainless steels, vanadium, molybdenum, copper, silver, manganese, platinum group elements, graphite, and chromium. The substrates may be made from an electrically conductive alloy of two or more metals or a mixture of two or more electrically conductive materials. A preferred material is nickel or nickel-based alloys. Nickel has a good stability in strong alkaline solutions, has a good conductivity and is relatively cheap.

The catalyst layer preferably is selected from nickel, cobalt, iron, or platinum group elements. The catalyst layer may include these elements as elemental metals, compounds (e.g. oxides), composite oxides or alloys made of multiple metal elements, or mixtures thereof. Preferred catalyst layers include plated nickel, plated alloys of nickel and cobalt or nickel and iron, complex oxides including nickel and cobalt such as LaNiO3, LaCoO3, and NiCo2O4, compounds of platinum group elements such as iridium oxide, or carbon materials such as graphene.

A particularly preferred catalyst layer comprises Raney Nickel. The Raney nickel structure is formed by selectively leaching aluminium or zinc from a Ni-Al or Ni-Zn alloy. Lattice vacancies formed during leaching result in a large surface area and a high density of lattice defects, which are active sites for the electrocatalytic reaction to take place.

Preferred porous electrodes and methods to prepare them are disclosed in for example EP-A 3575442, paragraphs 23 to 84.

The pore size of porous electrodes may have an influence on the electrolysis efficiency. For example, in EP-A 3575442 it is disclosed that preferred pore sizes of the porous electrodes are from 10 nm up to 200 nm.

The catalyst layer can also include organic substances such as polymers to improve the durability and the adhesion towards the substrate. The polymer in the catalyst layer is preferably similar to the polymer of the AEM membrane. Most preferably, the polymer in the catalyst layer is an amidinium-functionalized polymer. The amidinium-functionalized polymer of the catalyst layer is preferably provided in the form of a polymeric dispersion as referred to above.

The catalyst layer may also be provided on a surface of the membrane, resulting in a so-called Catalyst Coated Membrane (CCM). Such a CCM may have an improved contact surface between the membrane surface and the catalyst layer resulting in a higher electrolysis efficiency.

The catalyst layer may be applied on the membrane surface by any deposition technique such as coating, spraying, inkjet printing, gravure printing, screen printing, 3D printing, or vapour deposition techniques.

A typical AEM water electrolyser includes several electrolytic cells, also referred to as stack of electrolytic cells.

Regarding the cell configuration, two types of electrolysers are typically used.

A unipolar (or "tank- type") electrolyser consists of alternate positive and negative electrodes held apart by a membrane. Positive electrodes are all coupled together in parallel, as are the negative electrodes, and the whole assembly is immersed in a single electrolyte bath ("tank") to form a unit cell. A plant-scale electrolyser is then built up by connecting these units electrically in series. The total voltage applied to the whole electrolysis cell is the same as that applied to the individual unit cells.

On the other hand, in a bipolar electrolyser a metal sheet (or "bipole") connects electrically adjacent cells in series. The electrocatalyst for the negative electrode is coated on one face of the bipole and that for the positive electrode of the adjacent cell is coated on the reverse face. In this case, the total cell voltage is the sum of the individual unit cell voltages. Therefore, a series-connected stack of such cells forms a module that operates at a higher voltage and lower current than the tank-type (unipolar) design. To meet the requirements of a large electrolysis plant, these modules are connected in parallel to increase the current.

Membrane Electrode Assemblies (MEA) can also be used in an electrolyser. Such MEAs are typically prepared by applying a membrane, preferably without a reinforcing support, on at least one porous electrode. Such MEAs are for example disclosed in EP-A 2831312 (Agfa Gevaert), EP3277862 (De Nora) and WO2020/158719 (Nippon Shokubai). Such MEAs may also be used in the electrolysis method according to the present invention.

### Examples

### Materials

All materials used in the following examples were readily available from standard sources such as ALDRICH CHEMICAL Co. (Belgium) and ACROS (Belgium) unless otherwise specified. The water used was deionized water.

Proglyde DMM is dipropylene glycol dimethyl ether.

TEMPO is 2,2,6,6-Tetramethylpiperidin-1-oxyl radical.

WAKO V59 is 2,2'-Dimethyl-2,2'-azodibutyronitrile supplied by Wako Chemicals GmbH.

### Evaluation methods

### Molecular mass

The molecular mass was determined using TLC-MS, according to the following procedure. A TLC was run under circumstances given in the synthetic examples. The TLC was analyzed using a CAMAG ^{™} TLC-MS interface coupled to an AmaZon ^{™} SL mass spectrometer (supplied by Brüker Daltonics) via an Agilent ^{™} 1100 HPLC pump. First a blank spectrum was taken by eluting a spot on the TLC plate where no compounds are present with a 0.01 molar solution of ammonium acetate in methanol. A second spectrum of the compound to be analyzed was taken by eluting the spot of the compound under consideration with a 0.01 molar solution of ammonium acetate in methanol. The first spectrum was subtracted from the second spectrum, giving the spectrum of the compound to be analyzed.

### Example 1

An initial evaluation of the alkali-stability of the monomers and polymers according to the present invention was done on low molecular weight model compounds.

### Synthesis of model compound M1

Model compound M1, having a structure given below, is synthesized in 3 steps.

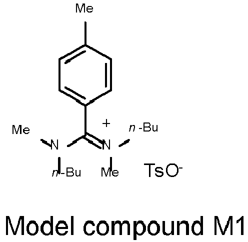

### Step 1: The synthesis of dibutyl carbodiimide

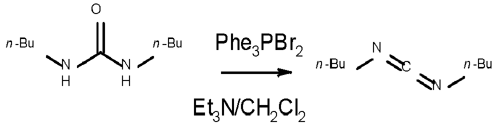

72.55 g (0.165 mol) dibromotriphenylphosphorane was dissolved in 190 mL methylene chloride. The reaction mixture was cooled to 0 °C and 34.1 g (0.333 mol) triethyl amine was added dropwise, while maintaining the temperature below 3 °C. A solution of 22.74 g (0.132 mol) dibutyl urea in 50 mL methylene chloride was added while maintaining the temperature at 0 °C. The reaction was allowed to continue for 90 minutes at 0 °C. 330 mL water was added to the mixture over 15 minutes, while maintaining the reaction temperature below 5°C. The mixture was stirred for an additional 15 minutes. The mixture was allowed to warm to room temperature and the methylene chloride layer was isolated. The methylene chloride layer was dried over MgSO₄ and evaporated under reduced pressure. 240 mL hexane was added to the residue and the residual triphenyl phosphine oxide was removed by filtration. The triphenyl phosphine oxide was treated a second time with 240 mL hexane. The pooled hexane fractions were evaporated under reduced pressure and dibutyl carbodiimide was purified by distillation (b.p. 82 °C at 11 mbar). 13.6 g (y : 67%) of dibutyl carbodiimide was isolated.

### Step 2: The addition of 4-tolyl magnesium bromide to dibutyl carbodiimide:

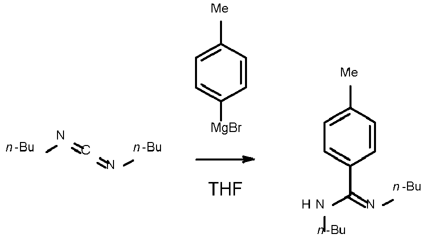

0.666 g (4.32 mmol) dibutyl carbodiimide was dissolved in 3 mL tetrahydrofuran. The reaction mixture was cooled to -5 °C. 4.32 mL of a 1M solution of p-tolyl magnesium bromide in tetrahydrofuran (4.32 mmol) was added over 145 minutes while keeping the temperature below 0°C. The reaction mixture was allowed to warm to room temperature and the reaction was allowed to continue for 16 hours at room temperature. The reaction mixture was cooled to -5 °C and an additional 0.864 mL of a 1 M solution of p-tolyl magnesium bromide in tetrahydrofuran (0.864 mmol) was added. The reaction mixture was allowed to warm to room temperature. The reaction was allowed to continue for 2 hours at room temperature. The reaction mixture was cooled to -5 °C and an additional 0.432 mL of a 1M solution of p-tolyl magnesium bromide in tetrahydrofuran (0.432 mmol) was added. The reaction mixture was allowed to warm to room temperature. The reaction was allowed to continue for 2 hours at room temperature. The reaction mixture was cooled to -5 °C and 3 mL methanol was added to the mixture while maintaining the reaction temperature below 5 °C. The precipitated salts were removed by filtration and washed with 20 mL methyl t.butyl ether. The salts were treated with 20 mL of a methylene chloride/methanol 1/1 mixture. The organic fraction were pooled and the solvents were removed under reduced pressure. 30 mL water was added to the residue and the pH was adjusted to pH=10 using a 10 N NaOH solution. The mixture was extracted with 100 mL methyl t.butyl ether. The methyl t.butyl ether fraction was isolated and the pH of the aqueous layer was adjusted to 12, using a 10 N NaOH solution. The aqueous layer was extracted again with 100 mL methyl t.butyl ether. The methyl t.butyl ether fractions were pooled, dried over MgSO₄ and evaporated under reduced pressure. The crude amidine was purified using preparative column chromatography on a Varian Mega Bond Elut C18 column, using methanol/0.2 M ammonium acetate 70/30 as eluent (TLC analysis on a Reveleris RP18 TLC plate, eluent methanol/1 M NaCl, R_{f} : 0.45). The pH of the pooled fractions was adjusted to 12, using a 10 N NaOH solution. The pooled fractions were extracted with 250 mL methyl t.butyl ether. The organic fraction was dried over MgSO₄ and evaporated under reduced pressure. 0.463 g (y : 39%) of the purified amidine was isolated.

### Step 3., The synthesis of model compound M1

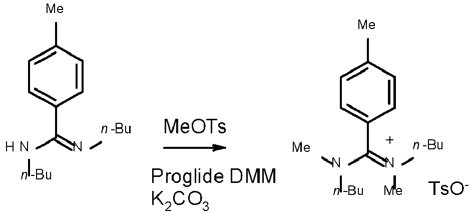

0.430 g (1,75 mmol) of the amidine was dissolved in 3 mL Proglyde DMM. 0.574 g (4.15 mmol) K₂CO₃ and 0.67 g (3.49 mmol) methyl tosylate were added and the reaction mixture was heated to 76°C. The reaction was allowed to continue for 17 hours at 76°C. After 17 hours an additional 0.326 g (1.75 mmol) methyl tosylate was added. The reaction was allowed to continue for an additional two hours at 76°C. The reaction mixture was allowed to cool down to room temperature. The precipitated salts were removed by filtration. The salts were washed with 7 mL methyl t.butyl ether. The organic fractions were pooled and the solvents were evaporated under reduced pressure. The residue was treated with 6 mL water and the pH was adjusted to 10, using a 10 N NaOH solution. The mixture was extracted with 30 mL methyl t.butyl ether. The aqueous fraction was isolated and extracted with 20 mL methylene chloride. The methylene chloride fraction was isolated, dried over MgSO₄ and evaporated under reduced pressure. 0.531 g (y : 64%) of model compound M1 was isolated (TLC analysis on Uniplate (Reveleris RP18), eluent methanol/1M NaCl 75/25, R_{f} : 0.39). The molecular mass of model compound M1 was confirmed using the TLC-MS methodology, as described above.

### Synthesis of model compound M2

Model compound M2, having a structure given below, was synthesized in 3 steps.

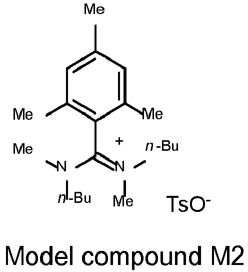

### Step 1: The synthesis of dibutyl carbodiimide

Dibutyl carbodiimide was prepared in the same way as for model compound M1 described above.

### Step 2: The addition of 2-mesityl magnesium bromide to dibutyl carbodiimide

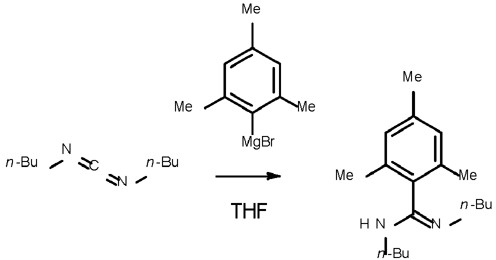

13.88 g (90 mmol) of dibutyl carbodiimide was dissolved in 45 mL tetrahydrofuran. The reaction mixture was cooled to -5°C. 90 mL of a 1M solution of 2-mesityl magnesium bromide in tetrahydrofuran (90 mmol) was added over 145 minutes, while maintaining the temperature below 0°C. The reaction mixture was allowed to warm to room temperature and the reaction was allowed to continue for 16 hours at room temperature. The reaction mixture was cooled to -5°C and 70 mL methanol was added to the mixture over 10 minutes. The precipitated salts were removed by filtration and washed with 50 mL of methyl t.butyl ether. The organic fractions were pooled, followed by the addition of 700 mL water. The pH was adjusted to 10.3, using a 10 NaOH solution. The mixture was extracted once with 1000 mL methyl t.butyl ether and once with 500 mL methyl t.butyl ether. The pooled organic fraction was extracted with 500 mL water and dried over MgSO₄. The solvent was removed under reduced pressure and 21.5 g of the crude the intermediate aminidine was isolated. The crude amidine was purified on a Varian Mega Bond Elut C18 column, using methanol/0.2 M ammonium acetate 70/30 as eluent (TLC analysis on a Reveleris RP18 TLC plate, eluent methanol/1 M NaCl 80/20 , R_{f} : 0.51). The pH of the pooled fractions was adjusted to 12, using a 10 N NaOH solution. The pooled fractions were extracted with 250 mL methyl t.butyl ether. The organic fraction was dried over MgSO₄ and evaporated under reduced pressure. 17.8 g (y : 72%) of the purified amidine was isolated.

### Step 3., The alkylation with methyl tosylate and the synthesis of model compound M2:

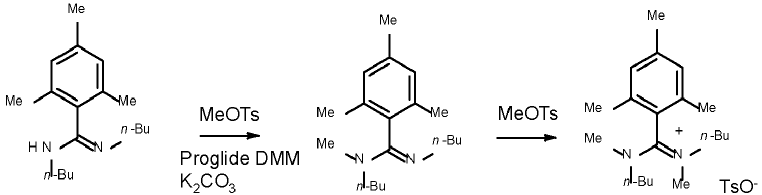

5.49 g (20 mmol) of the amidine was dissolved in 28 mL Proglyde DMM. 6.58 g (47.6 mmol) K₂CO₃ and 4.22 g (22 mmol) methyl tosylate were added and the reaction mixture was heated to 76 °C. The reaction was allowed to continue for 17 hours at 76 °C. The reaction mixture was allowed to cool down to room temperature. The salts were removed by filtration and washed with 80 mL methyl t.butyl ether. The organic fractions were pooled and the solvents were removed under reduced pressure. 60 mL water was added to the residue and the pH was adjusted to 12, using a 10 N NaOH solution. The aqueous layer was extracted with 320 mL methyl t.butyl ether. The organic fraction was dried over MgSO₄ and evaporated under reduced pressure. 4.42 g (y : 77%) of the alkylated amidine was isolated (TLC analysis on a Reveleris RP18 TLC plate, eluent methanol/1 M NaCl 75/25 , R_{f} : 0.33, TCL-MS analysis according to the method described above : MM = 288). The aqueous layer was extracted a second time but now with 200 mL methylene chloride. The organic fraction was isolated, dried over MgSO₄ and evaporated under reduced pressure. 1.4428 g of the amidinium model compound M2 was isolated (TLC analysis on a Reveleris RP18 TLC plate, eluent methanol/1 M NaCl 75/25, R_{f} : 0.33, TCL-MS analysis according to the method described above : MM = 303, which corresponds to the cation of model compound M2).

### Synthesis of model compound M3

Model compound 3 was prepared in order to compare the stability of the cationic moieties according to the present invention in alkaline medium to the stability of other highly stable cationic moieties disclosed in the prior art. The functional groups disclosed by Fan et al. (ACS Macro Letters, 6(10), 1089-1093 (2017)) were selected for this purpose. A model compound M3, having the same substituents and counterion as model compound M2 was prepared.

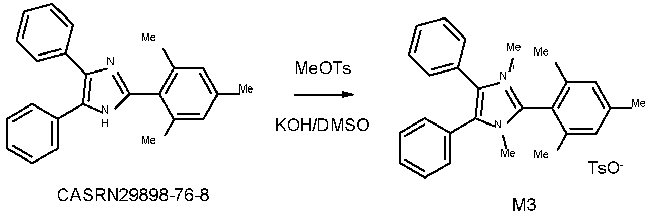

CASRN29898-78-8 was prepared as disclosed by Fan et al. (ACS Macro Letters, 6(10), 1089-1093 (2017)).

CASRN29898-78-8 was then converted into model compound M3 according to the following procedure:
0.337 g (6 mmol) potassium hydroxide was dissolved in 15 mL DMSO.
1.015 g of CASRN29898-76-8 (3 mmol) was dissolved in 15 mL DMSO
and added to the potassium hydroxide solution over 5 minutes. The mixture was stirred for one hour at room temperature. 0.593 g (3.12 mmol) methyl tosylate was added and the reaction was allowed to continue for one hour at room temperature. The reaction was monitored using TLC chromatography on a Merck Silica gel 60F₂₅₄ TLC-plate, using n-hexane/ethyl acetate 80/20 as eluent. After one hour, CASRN29898-78-8 was completely converted in the N-methylated compound (CASRN2131737-37-4, R_{f} : 0.38 ; TLC-MS analysis : mm = 352). The reaction mixture was added to 240 mL water containing 0.6 g potassium hydroxide. The mixture was extracted with 90 mL dibutyl ether. The organic fraction was washed with 50 mL water, 50 mL brine and again with 50 mL water. The organic fraction was dried over MgSO₄ and evaporated under reduced pressure. 1.019 g of CASRN2131737-37-4 was isolated and dissolved in 15 mL methylene chloride. 2.394 g (12.6 mmol) methyl tosylate was added and the reaction was allowed to continue for 18 hours at 30 °C. The solvent was removed under reduced pressure and model compound M3 was purified, using preparative column chromatography on a Prochrom LC80 column, using Kromasil C18 100A 10 µm as stationary phase and methanol/ 0.2 M ammonium acetate 70/30 as eluent. 0.823 g (y : 51%) of model compound M3 was isolated (TLC analysis on Whatman Partisil KC18F, methanol/1 M NaCl 70/30 as eluent: R_{f} : 0.42; TLC MS analysis : mm of the cation in positive mode : 367).

### Stability of model compounds M1, M2, and M3 in alkaline solution

To compare the stability of model compounds M1 and M2 in alkaline medium, a 1 w% solution of both compounds was made in 2 M KOH. The mixture was stored at 80 °C and the stability of the compounds was analyzed with TLC chromatography on a Uniplate Reveleris Rp18 plate, using methanol/1 M NaCl 80/20 as eluent. To further confirm the stability, a TLC-MS analysis was done on each sample to confirm the structure. The first sample was taken after 24 hours at 80 °C.

After 24 hours, the model compound M1 was completely degraded with the degradation compounds D1 and D2 being formed, optionally in ionic form:

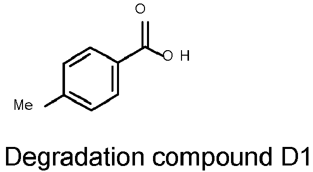

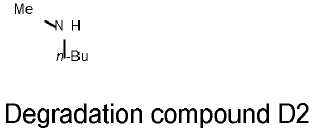

The model compound M2 remained completely intact even after 18 days at 80 °C. No trace of any degradation product could be found. The integrity of the structure was further proven by TLC-MS. No trace of e.g. dealkylation could be found, proving the excellent alkaline stability of the compounds according to the present invention.

To compare the stability of model compounds M2 and M3, A solution of 2 w% of both compounds in 2M potassium hydroxide was prepared. The solutions were stored at 80 °C for 17 days and re-analyzed using TLC chromatography combined with mass spectroscopy (TLC analysis on Whatman Partisil KC18F, eluent: methanol/1 M NaCl 70/30)

Model compound M2 proved to be completely stable over 17 days at 80 °C, while a side degradation product was detectable for model compound M3 (R_{f} : 0.065). The structure of the degradation product was elucidated using mass spectroscopy and proved to be CASRN2131737-37-4.

CASRN2131737-37-4 was formed by de-alkylation of model compound M3 under the experimental conditions used as illustrated in the scheme below.

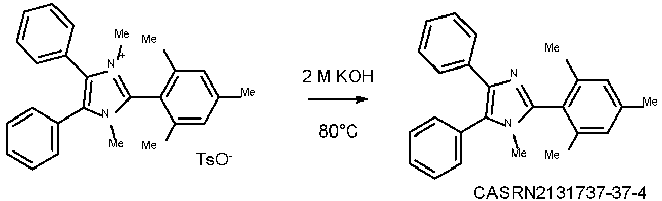

From the experiments above, it can be concluded that the cationic compounds according to the present invention show an improved stability in alkaline medium compared to state-of-the-art cationic moieties, disclosed in the prior art.

### Example 2

An amidinium-functionalized monomer AM-1 was synthesized starting from an amidine-functionalized precursor molecule APM-1. The alkaline stability of AM-1 was subsequently evaluated.

### Synthesis of amidine-functionalized precursor monomer APM-1

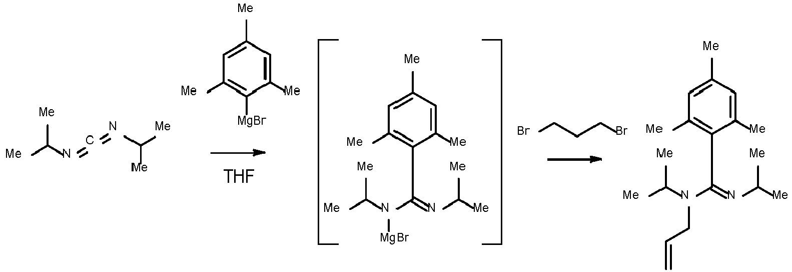

1.28 g (10 mmol) N,N-diisoprpyl carbodiimide was dissolved in 1 mL tetrathydrofuran. 12 mL of a 1 M solution of 2-mesityl magnesium bromide in THF (12 mmol) was added over 90 minutes. The reaction temperature increased to 43 °C during the addition. The reaction was allowed to continue for an additional 30 minutes at room temperature. 2.243 g (11 mmol) 1,3-dibromo-propane was added and the reaction mixture was refluxed for two hours. The solvent was removed under reduced pressure and 6 mL dimethyl formamide was added to the reaction mixture. 1.514 g (10 mmol) sodium iodide and 2.43 g (11 mmol) dibromopropane were added and the reaction mixture was heated to 120 °C. The reaction was allowed to continue for 1 hour. The solvent was removed under reduced pressure and the amidine was isolated using preparative column chromatography on a Prochrom LC80 column, using Kromasil C18 100A, 10 µm as stationary phase and methanol/0.2 M ammonium acetate 70/30 as eluent. 0.25 g (y : 8.7 %) of the amidine was isolated (TLC analysis on Reveleris RP18 TLC plate, eluent methanol/1 M NaCl 70/30 , R_{f} : 0.26, TLC-MS analysis according to the method described above : MM : 286).

### Synthesis of amidinium-functionalized monomer AM-1

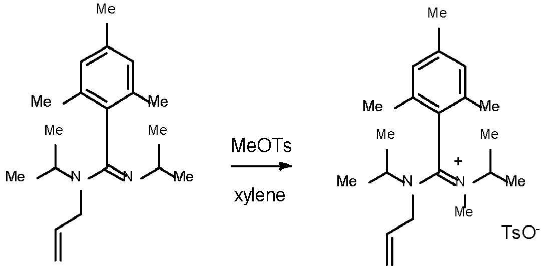

0.211 g (0.74 mmol) of the amidine was dissolved in 1 mL xylene. 0.435 g (2.26 mmol) methyl tosylate was added and the reaction mixture was heated to 135 °C. The reaction was allowed to continue for 6 hours at 135 °C. The reaction mixture was allowed to cool down to room temperature and AM-1 phase separated from the medium. The xylene was removed and the residue was dried under reduced pressure. The residue was redissolved in 1 mL methylene chloride and precipitated with methyl t.butyl ether. AM-1 precipitated as an oil. The solvents were removed and the residue was dried under reduced pressure. 0.245 g (y : 70%) of AM-1 was isolated (TLC analysis on a Whatman RP18 plate, eluent methanol/1 M NaCL 70/30 : R_{f} : 0.42; TLC-MS analysis according to the method described above : MM : 301, corresponding to the cation of AM-1).

### Stability of AM-1 in alkaline solution

A 1 w% solution of AM-1 in 2 M KOH was prepared and the solution was stored at 80 °C. The stability was analyzed using TLC-analysis on a Whatman RP18 plate, using methanol/1 M NaCl as eluent. Each sample was analyzed by TLC-MS analysis according to the method described above. After 47 days at 80 °C no trace of degradation could be detected. The TLC-MS analysis confirmed the structural integrity of AM-1, proving the excellent alkaline stability of the compounds according to the present invention.

### Example 3

An amidine-functionalized precursor monomer APM-2 was synthesized and polymerized to an amidine-functionalized polymer APP-1, which was then quaternized with the formation of amidinium-functionalized polymer AP-1.

### Synthesis of amidine-functionalized precursor monomer APM-2

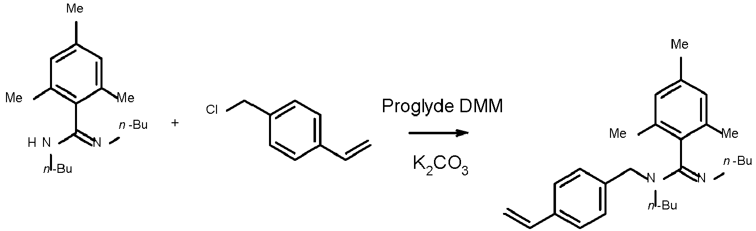

2.744 g (10 mmol) of the above described amidine was dissolved in 12 mL Proglyde DMM. 2.764 g (20 mmol) K₂CO₃ was added followed by the addition of 0.062 g TEMPO and 1.526 g (10 mmol) 4-vinyl-benzyl chloride. The reaction mixture was heated to 120°C and the reaction was allowed to continue for 17 hours at 120°C. The reaction mixture was allowed to cool down to room temperature and the salts were removed by filtration. The salts were washed with 20 mL methanol and the pooled organic fractions were evaporated under reduced pressure. 20 mL water was added to the oily residue and the pH was adjusted to 12 using a 10 N NaOH solution. The mixture was extracted with 60 mL methyl t.butyl ether. The organic fraction was isolated, dried over MgSO₄ and evaporated under reduced pressure. APM-2 was purified by preparative column chromatography on a Varian Mega Bond Elut C18 column, using methanol/0.2 M ammonium acetate 70/30 as eluent. 2.26 g (y : 58 %) of APM-2 was isolated (TLC analysis on Reveleris RP18 TLC plate, eluent methanol/1 M NaCl 80/20 , R_{f} : 0.36, TLC-MS analysis according to the method described above : MM : 390)

### Synthesis of amidine-functionalized precursor polymer APP-1

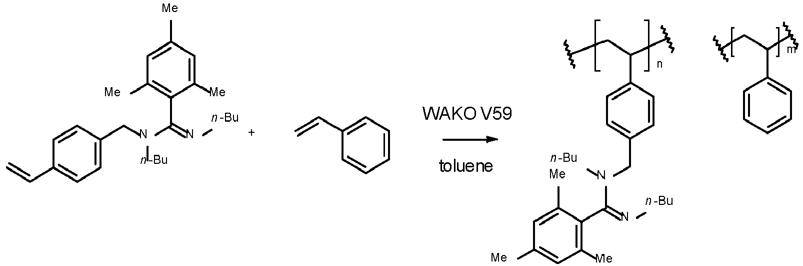

1 g (2.56 mmol) of the styrene functionalized amidine was dissolved in 5 g toluene. 1 g (9.6 mmol) styrene was added. A solution of 23 mg (0.12 mmol) WAKO V59 in 1 g toluene was prepared. The reaction mixture was heated to 83°C and the toluene solution of WAKO V59 was injected into the reaction mixture. The needle was rinsed additionally with 1 g toluene. The polymerization was allowed to continue for 2 hours at 83 °C. An additional 23 mg (0.12 mmol) WAKO V59 in 1 g toluene was added and the polymerization was allowed to continue for 17 hours at 83°C. An additional 12 mg (0.06 mmol) WAKO V59 in 1 g toluene was added and the reaction mixture was heated to 93 °C. The polymerization was allowed to continue for an additional two hours. The conversion of the monomer was monitored using TLC chromatography (TLC analysis on Reveleris RP18 TLC plate, eluent methanol/1 M NaCl 80/20). After the third addition only traces of residual monomer were detectable. The reaction mixture was allowed to cool down to room temperature and the solvent was removed under reduced pressure. 20 mL methanol was added to the residue upon which APP-1 precipitated. APP-1 was isolated by filtration. The isolated polymer was treated with 30 mL ethanol and isolated again by filtration. 1.32 g (y : 66%) of APP-1 was isolated.

### Synthesis of amidinium-functionalized polymer AP-1

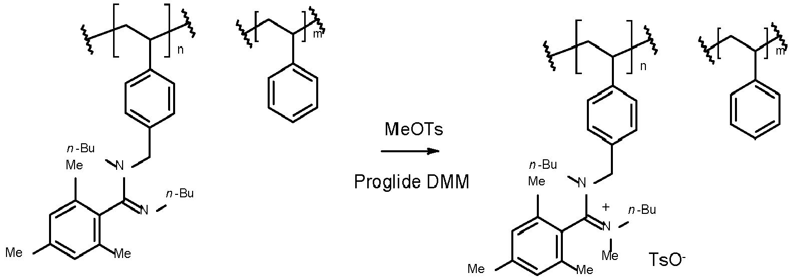

1.160 g of APP-1 was dissolved in 4 mL Proglyde DMM. A solution of 0.2 g (1.04 mmol) methyl tosylate in 1 mL Proglyde DMM was added and the reaction mixture was heated to 125 °C. The conversion of the polymer was monitored by TLC analysis ((TLC analysis on Reveleris RP18 TLC plate, eluent methanol/1 M NaCl 70/30). After 16 hours at 125 °C, no methyl tosylate was detectable anymore. The tosylate counterion of the polymer could clearly be detected. The structure of the counterion was proven by TLC-MS, according to the method described above. An additional 0.2 g ((1.04 mmol) methyl tosylate was added and the reaction was allowed to continue for 5 hours at 125 °C. Upon TLC analysis, traces of methyl tosylate could be detected, which did not convert any further. The reaction mixture was allowed to cool down to room temperature upon which the polymer separated from the reaction mixture. The solvent was removed and the residue was dried under reduced pressure. The residue was treated with 50 mL methyl t.butyl ether, upon which the polymer solidified. The polymer was isolated and dried. 1.06 g of AP-1 was isolated.

### Example 4

Amidinium-functionalized monomer AM-2 was synthesized starting from APM-2 and subsequently polymerized to form an amidinium-functionalized polymer AP-1' having the same chemical formula as AP-1. This example shows that there are different methods to obtain an amidinium-functionalized polymer.

### Synthesis of amidinium-functionalized monomer AM-2

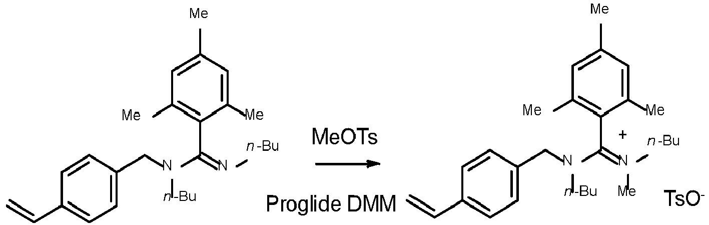

0.55 g (1.41 mmol) APM-1 was dissolved in 5 mL Proglyde DMM. 9 mg TEMPO was added followed by the addition of 0.551 g (2.82 mmol) methyl tosylate. The reaction mixture was heated to 120 °C and the reaction was allowed to continue for 150 minutes at 120 °C. The reaction mixture was allowed to cool down to room temperature and the solvent was removed under reduced pressure. 25 mL water was added to the oily residue and the pH was adjusted to 12 using a 10 N NaOH solution. The mixture was extracted with 50 mL methyl t.butyl ether. The methyl t.butyl ether fraction was extracted three times with 25 mL water, adjusted to an alkaline pH by adding 400 µl of a 10 N NaOH solution. The pooled water fractions were extracted with 200 mL methylene chloride. The methylene chloride fraction was isolated, dried over MgSO₄ and evaporated under reduced pressure. 0.37 g (y : 46%) of AM-2 was isolated (TLC analysis on Reveleris RP18 TLC plate, eluent methanol/1 M NaCl 80/20 : R_{f} : 0.35; TLC-MS analysis according to the method described above : MM for the cation : 405).

### Synthesis of amidinium-functionalized polymer AP-1' by polymerization of the amidinium-functionalized monomer AM-2

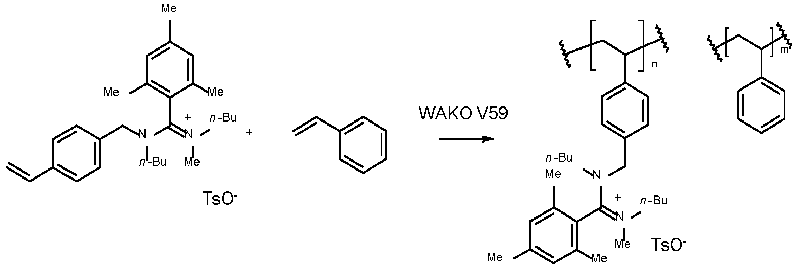

0.35 g (0.61 mmol) AM-2 and 0.35 g (3.36 mmol) styrene were dissolved in 4.5 mL toluene. A nitrogen flow was put over the reaction mixture to remove oxygen. 12 mg (0.06 mmol) WAKO V59, dissolved in 0.5 mL toluene, was added and the reaction mixture was heated to 83 °C, while stirring. The reaction was allowed to continue for 2 hours at 83 °C. After 2 hours, an additional 12 mg (0.06 mmol) WAKO V59 in 0.5 mL toluene was added. The polymerization was allowed to continue for 21 hours at 83°C. The reaction was monitored, using TLC chromatography (TLC analysis on Reveleris RP18 TLC plate, eluent methanol/1M NaCl 82/20, R_{f} of AM-2 : 0.35). After 21 hours at 83 °C, AM-2 was completely converted to AP-1'. The solvent was removed under reduced pressure and the residue was treated first with 20 mL methyl t.butyl ether followed by treating the residue with 30 mL ethanol. Upon treatment with ethanol, AP-1' solidified and was isolated by filtration; 0.305 g (y : 44%) of AP-1' was isolated.

## Claims

1. An amidinium-functionalized compound, **characterized in that** the compound has a structure according to General Formula I or General Formula II 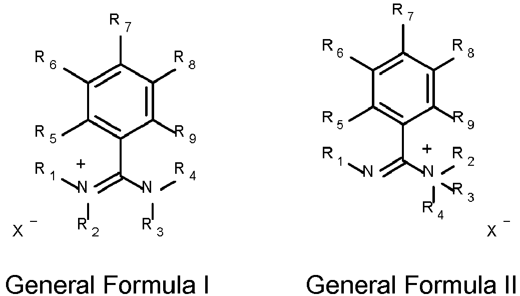 wherein
• R₅ and R₉ are any substituent different from hydrogen;
• R₁ to R₄ are independently selected from the group consisting of an alkyl group, an alkenyl group, an alkynyl group, an aralkyl group, an alkaryl group, an aryl group and a heteroaryl group, or any of R₁ and R₃, R₁ and R₄, R₁ and R₂, R₃ and R₄, R₂ and R₃, or R₂ and R₄ represent the necessary atoms to form a five- to eight- membered non-aromatic ring;
• R₆ to R₈ are independently selected from the group consisting of hydrogen, an alkyl group, an alkenyl group, an alkynyl group, an aralkyl group, an alkaryl group, an aryl or heteroaryl group, a halogen group, an ether group, a nitro group, an amine group, or any of R₅ and R₆, R₆ and R₇, R₇ and R₈, or R₈ and R₉ represent the necessary atoms to form a fiveto eight-membered ring;
• X- is an anion;
and wherein
• at least one of R₁ to R₉ comprises a polymerizable group or comprises the necessary atoms to link the amidinium group to a polymer.

2. The compound according to claim 1, wherein X- is selected from the group consisting of iodide, bromide, chloride, fluoride, triiodide, hydroxide, carbonate, bicarbonate, cyanide, acetate, nitrate, sulfate, alkyl sulfate, perfluorinated alkylsulfonate, arylsulfonate, perchlorate, tetrachloroaluminate, tetrafluoroborate, alkyl borate, phosphate, halophosphate, alkyl phosphate, triflate, tosylate, mesylate, alkyl carboxylate, tetrakis(3,5-bis(trifluoromethyl)phenyl)borate, bis(trifluoromethane)sulfonamide, and any combination thereof.

3. The amidinium-functionalized compound according to claim 1 or 2, wherein R₅ and R₉ are independently selected from an alkyl group and an aryl group.

4. The amidinium-functionalized compound according to claim 3, wherein R₅ and R₉ are independently selected from a methyl group and a phenyl group.

5. The amidinium-functionalized compound according to any of the claims 1 to 4, wherein at least one of R₁ to R₉ comprises a polymerizable group.

6. The amidinium-functionalized compound according to claim 5, wherein at least one of R₁, R₂, R₃, R₄ or R₇ comprises a polymerizable group selected from styrene, epoxy, oxetane, vinyl ether, or alkene.

7. A polymer comprising a monomeric unit of the amidinium-functionalized compound of claim 5 or 6.

8. The polymer according to claim 7, wherein the amidinium group of the amidinium functionalized compound forms part of the polymer backbone.

9. A dispersion comprising the polymer according to claim 7 or 8.

10. An anion exchange membrane comprising the amidinium-functionalized polymer as defined in claims 7 or 8.

11. The anion exchange membrane according to claim 10, coated with an electrocatalyst selected from nickel, molybdenum, copper, iron, ruthenium, iridium, or platinum.

12. The anion exchange membrane according to claim 11, wherein the coating further comprises the polymer according to claim 7 or 8.

13. An electrochemical device comprising the anion exchange membrane according to any of the claims 10 to 12.

14. The electrochemical device according to claim 13, wherein the device is a water electrolyser, a fuel cell, a CO₂-electrolyser, an electrodialyser, or a redox-flow battery.

15. A method for making the polymer according to claim 7 or 8, comprising:
i. Polymerizing an amidine-functionalized precursor compound according to General Formula III 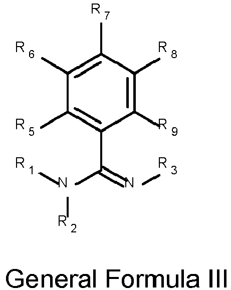 wherein R₁ to R₉ are as defined in claim 5 or 6;
ii. Performing a quaternization step with an alkylating agent to obtain a cationic polymer;
or
i.' Polymerizing the amidinium-functionalized compound according to claim 5 or 6.

16. Use of the anion exchange membrane according to any of the claims 10 to 12 to produce green hydrogen, green ammonia, or green steel.
